# EUROPÄISCHE PATENTSCHRIFT

(11) **EP 0 566 850 B1**
(45) Veröffentlichungstag und Bekanntmachung des Hinweises auf die Patenterteilung: **11.09.1996**
(21) Anmeldenummer: 93103692.5
(22) Anmeldetag: 08.03.1993
(51) Int. Cl.: A61N 1/05

(54) **Steuerbare Elektrodenvorrichtung**
Controllable electrode device
Dispositif d'électrodes dirigeable

(30) Priorität: 24.04.1992 SE 9201295
(43) Veröffentlichungstag der Anmeldung: 27.10.1993
(73) Patentinhaber: Pacesetter AB, 171 95 Solna (SE)
(72) Erfinder: Nyman, Per, S-182 64 Djursholm (SE)
(74) Vertreter: Lettström, Richard Wilhelm

(56) Entgegenhaltungen:
- EP-A- 0 336 865
- US-A- 4 136 703
- US-A- 4 257 429
- US-A- 4 401 127
- US-A- 4 402 328

## Beschreibung

Die Erfindung betrifft eine steuerbare Elektrodenvorrichtung für die intrakardiale Stimulation des Herzens mit einem Elektrodenkabel, das einen langgestreckten, flexiblen Leiter umfasst, dessen Aussenseite mit einer Isolierschicht versehen ist und dessen Innenseite einen Kanal zur Einführung eines Steuermittels bildet, und mit einem Elektrodenkopf, der am distalen Ende des Leiters zur Stimulierung des Herzgewebes angebracht ist.

Es ist von grosser Bedeutung, dass das Elektrodenkabel bei einer derartigen Elektrodenvorrichtung ausreichend weich ist, damit das Kabel bei der Einführung in das Herz eines Patienten über eine Vene dieser folgen kann, ohne dass die umgebende Venenwand beschädigt wird. Die Einführung des Elektrodenkabels erfolgt in den meisten Fällen mit Hilfe eines Mandrins, der in den Kanal des Kabels eingeschoben wird und der aus einem Material hergestellt ist, das ausreichend steif ist, um das Elektrodenkabel durch die Vene vorschieben zu können. Die Steifheit des Elektrodenkabels kann daher abhängig vom Durchmesser und Material des Mandrins variiert werden. Bei schwierigen Passagen, bei denen das Elektrodenkabel z.B. wesentlich gebogen werden muss, wird der Mandrin eine Strecke zurückgezogen, so dass das distale Ende des Elektrodenkabels maximal weich wird. Nachdem eine solche Passage passiert worden ist, wird der Mandrin wieder bis zum distalen Ende des Elektrodenkabels vorgeschoben, damit dieses Ende zum Vorhof oder zur Kammer des Herzens transportiert wird, so dass der Elektrodenkopf gegen eine Herzwand zur Stimulierung angelegt werden kann.

In der US-A-4 402 328 ist eine Elektrodenvorrichtung der eingangs genannten Art mit einem J-förmig vorgeformten Elektrodenkabel beschrieben, dessen Elektrodenkopf dafür vorgesehen ist, im Aurikel appliziert zu werden. Bei der Einführung des Elektrodenkabels in das Herz ist dieses mit Hilfe eines verhältnismässig steifen Mandrins begradigt. Aufgrund des steifen Mandrins kann es schwer sein, gewisse Venenpassagen vorbeizukommen.

Eine weitere entsprechende Elektrodenvorrichtung ist durch die US-A-4 136 703 bekannt. Bei dieser Elektrodenvorrichtung ist in den Kanal des Elektrodenkabels ein verhältnismässig steifes Rohr, das sich über die ganze Länge des Elektrodenkabels erstreckt, einschiebbar. In das Rohr ist ein J-förmig vorgeformter Mandrin eingeschoben, der mit Hilfe des Rohres begradigt ist. Wenn das distale Ende des Elektrodenkabels sich im Inneren des Herzens befindet, wird das Rohr nach hinten verschoben, so dass der Mandrin freigelegt wird, der dann das distale Ende des Elektrodenkabels J-förmig verbiegt. Ein Elektrodenkabel dieser Art ist extrem steif.

Der Erfindung liegt die Aufgabe zugrunde, eine Elektrodenvorrichtung der eingangs genannten Art mit einem Elektrodenkabel zu schaffen, das zu mindest im Bereich dessen distalen Endes weich und flexibel ist, das beim Verschieben in eine Vene steuerbar ist und das auch im Herzen in gewünschter Weise gesteuert und geformt werden kann.

Diese Aufgabe ist erfindungsgemäss dadurch gelöst, dass das Steuermittel ein langgestrecktes Element ist, das mit mindestens einer Kerbe versehen ist, die etwa senkrecht zur Längsrichtung des Elements verläuft und die eine derartige Form und Tiefe aufweist, dass das Element und somit auch das Elektrodenkabel im Bereich der Kerbe verbogen werden, wenn das Element einem Druck in seiner Längsrichtung ausgesetzt wird. Das langgestreckte Element kann ein Draht sein, der am distalen Ende des Elektrodenkabels anliegt und der mit Hilfe eines Mandrins einem Druck in seiner Längsrichtung ausgesetzt wird. Der Draht wird nun einem Druck ausgesetzt, der von der Biegung des Elektrodenkabels, die erreicht werden soll, abhängt. Hierdurch kann das distale Ende des Elektrodenkabels derart gesteuert werden, dass es leicht über eine Vene in das Herz geführt werden kann, um dort in gewünschter Weise geformt zu werden.

In einer vorteilhaften Weiterbildung der Erfindung wird vorgeschlagen, dass das langgestreckte Element mit einer Anzahl nacheinander angebrachter Kerben versehen ist, die ein im Profil sägezahnförmiges Teil bilden. Hierdurch ist die Möglichkeit gegeben, eine weiche Biegung des Elements und daher auch eine weiche Biegung des Elektrodenkabels zu erhalten.

Im Hinblick auf eine günstige konstruktive Ausgestaltung der Erfindung empfiehlt es sich, dass das langgestreckte Element ein Teil eines Mandrins ist.

In einer vorteilhaften Ausbildung der Erfindung wird vorgeschlagen,dass das langgestreckte Element elastisch ist, so dass es seine ursprüngliche Form einnimmt, wenn der Druck abnimmt. Dies ist von Vorteil, wenn das Elektrodenkabel in einer Vene gesteuert werden soll und wenn der Elektrodenkopf zwischen das Gewebe des Trabekelgewebes der Herzwand der Kammer eindringen soll, um eine gute Fixierung an die Herzwand sicherzustellen. Das Eindringen wird daruch erleichtert, daß das distale Ende des Elektrodenkabels abwechselnd gebogen und gestreckt und eventuell das Elektrodenkabel um seine Längsachse gedreht wird.

Eine weitere günstige Ausgestaltung der Erfindung ergibt sich, wenn das langgestreckte Element ein derartiges Material aufweist, dass es die gebogene Form beibehält, wenn der Druck abnimmt. Dies kann von Vorteil sein, wenn der Elektrodenkopf am Aurikel appliziert werden soll. In diesem Fall muss das distale Ende des Elektrodenkabels J-förmig gebogen werden und diese Form beibehalten.

Weitere Vorteile und Einzelheiten der Erfindung ergeben sich aus den abhängigen Ansprüchen.

Die Erfindung ist nachfolgend anhand eines in den Zeichnungen dargestellten Ausführungsbeispiels näher erläutert. Es zeigen:

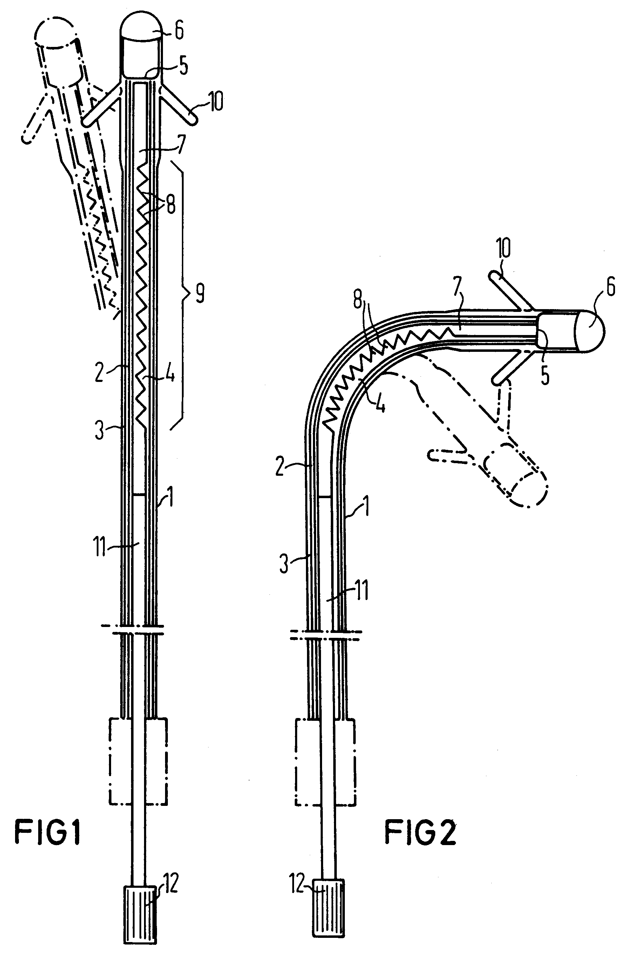

- FIG 1 und 2: Seitenansichten einer Elektrodenvorrichtung nach der Erfindung.

In der FIG 1 ist eine Elektrodenvorrichtung zur intrakardialen Stimulation eines Herzens abgebildet. Die Elektrodenvorrichtung, die zum grössten Teil im Schnitt gezeigt ist, besteht aus einem Elektrodenkabel 1, das einen langgestreckten flexiblen Leiter 2 umfasst, dessen Aussenseite mit einer Isolierschicht 3 versehen ist und dessen Innenseite einen Kanal 4 bildet. Am distalen Ende 5 des Leiters 2 ist ein Elektrodenkopf 6 zur Stimulierung des Herzgewebes eines Patienten angebracht. Dicht hinter dem Elektrodenkopf 6 sind Mittel 10 zum Fixieren des Elektrodenkopfes 6 angeordnet. Im Kanal 4 ist ein langgestrecktes Element in Form eines Drahts 7 eingeführt, der aus einem elastischen Material hergestellt ist. Der Draht 7 ist mit einer Anzahl nacheinander angebrachten Kerben 8 versehen,die ein im Profil sägezahnförmiges Teil 9 bilden. Der Draht 7 mit dem sägezahnförmigen Teil 9 ist am vorderen Teil des Elektrodenkabels 1 angebracht. Die Kerben 8 sind vorzugsweise V- und U-förmig ausgebildet.

Die Einführung des Elektrodenkabels 1 in eine Vene eines Patienten erfolgt mittels eines Mandrins 11, der in den Kanal 4 des Elektrodenkabels 1 eingeschoben wird,so dass das sehr weiche Elektrodenkabel 1 etwas steifer wird. Der Mandrin 11 wird dabei so weit vorgeschoben, dass dessen Endseite gegen die Endseite des Drahts 7 anliegt. Beim Drücken des Mandrins 11 gegen den Draht 7 werden die Kerben 8 derart deformiert, dass der Draht 7 und somit auch das Elektrodenkabel 1 in der in der FIG 2 gezeigten Weise gebogen werden. Wenn der Druck nachlässt, geht der Draht 7 sowie das Elektrodenkabel 1 in die ursprüngliche Lage zurück. Mit Hilfe des Mandrins 11 kann demnach der Draht 7 einem Druck, der von der Biegung des Elektrodenkabels, die erreicht werden soll abhängt, ausgesetzt werden. Durch Drehen des Elektrodenkabels 1 um seine Längsachse kann das Kabel 1 in jede gewünschte Richtung gebogen werden. Eine mögliche Biegung ist in der in der FIG 1 strichpunktierten Fassung des distalen Endes des Elektrodenkabels 1 dargestellt. Da der Draht 7 in dem Kanal 4 drehbar angebracht sein kann, kann er gegenüber dem Elektrodenkabel 1 um seine Längssachse derart gedreht werden, dass, wie bereits beschrieben, eine Biegung des Elektrodenkabels 1 erfolgt. In den Fällen, in denen der Draht 7 in dem Kanal 4 gedreht werden soll, ist der Mandrin 11 mit dem Draht 7 fest verbunden. Der Draht 7 kann auch ein Teil des Mandrins 11 sein. Durch Drehen des Griffs 12 des Mandrins 11 kann das sägezahnförmige Teil 9 und damit auch das distale Ende des Elektrodenkabels 1 in gewünschter Weise gesteuert werden. Somit kann das Elektrodenkabel 1 über eine Vene in das Herz gesteuert werden. Wenn das distale Ende des Elektrodenkabels 1 das Trabekelgewebe der Herzwand erreicht, kann der Elektrodenkopf 6 durch ein abwechselndes Biegen und Strecken des erwähnten Endes und eventuell durch ein gleichzeitiges Routieren des Elektrodenkabels 1 um seine Längsachse das Hindernis durchdringen, um die Herzwand zu erreichen. Die Fixiermittel 10 vermeiden eine Dislokation des Elektrodenkopfes 6.

Wenn der Elektrodenkopf 6 der Elektrodenvorrichtung am Aurikel plaziert werden soll, wird der Draht 7, wenn das distale Ende des Elektrodenkabels 1 den Vorhof erreicht hat, in der beschriebenen Weise zusammengedrückt, so dass das Ende J-förmig ausgebildet wird. Die in der FIG 2 strichpunktierte Version des distalen Endes des Elektrodenkabels 1 zeigt eine solche J-Form. Bei einer solchen Elektrodenvorrichtung kann der Draht 7 aus einem derartigen Material sein, dass er die gebogene Form beibehält, wenn der Druck vom Mandrin 11 abnimmt.

Der Draht 7 sollte derart ausgebildet sein, dass er ohne Druckbelastung dem Elektrodenkabel 1 eine minimale Erhöhung der Steifigkeit quer zur Längsrichtung gibt und keinen Verschleiss an den Leitern hervorruft. Durch den Draht 7 wird, abhängig von der Materialwahl und der Form der Kerben 8, eine gewünschte Flexibilität des Elektrodenkabels 1 im Hinblick auf die Steifigkeit quer zu dessen Längsrichtung erhalten, so dass dessen distalen Ende geschmeidig und anpassungsfähig ist. Auf diese Weise kann das Elektrodenkabel 1 durch eine Vene gesteuert werden, ohne dass das Kabel in einer scharfen Venenkurve hart gegen die Venenwand zu liegen kommt. Hierdurch ist das Risiko, die Venenwand zu penetrieren, minimal.

## Patentansprüche

1. Steuerbare Elektrodenvorrichtung für die intrakardiale Stimulation des Herzens mit einem Steuermittel und einem Elektrodenkabel (1), das einen langgestreckten, flexiblen Leiter (2) umfasst, dessen Aussenseite mit einer Isolierschicht (3) versehen ist und dessen Innenseite einen Kanal (4) zur Einführung des Steuermittels bildet, und mit einem Elektrodenkopf (6), der am distalen Ende des Leiters (2) zur Stimulierung des Herzgewebes angebracht ist, **dadurch gekennzeichnet**, dass das Steuermittel ein langgestrecktes Element (7) ist, das mit mindestens einer Kerbe (8) versehen ist, die etwa senkrecht zur Längsrichtung des Elements (7) verläuft und die eine derartige Form und Tiefe aufweist, dass das Element (7) und somit auch das Elektrodenkabel (1) im Bereich der Kerbe (8) verbogen werden, wenn das Element (7) einem Druck in seiner Längsrichtung ausgesetzt wird.

2. Steuerbare Elektrodenvorrichtung nach Anspruch 1, **dadurch gekennzeichnet,** dass das langgestreckte Element (7) mit einer Anzahl nacheinander angebrachter Kerben (8) versehen ist, die ein im Profil sägezahnförmiges Teil (9) bilden.

3. Steuerbare Elektrodenvorrichtung nach Anspruch 1 oder 2, **dadurch gekennzeichnet**, dass das sägezahnförmige Teil (9) am vorderen Teil des Elektrodenkabels (1) anliegt.

4. Steuerbare Elektrodenvorrichtung nach einem der Ansprüche 1 bis 3**, dadurch gekennzeichnet**, dass das langgestreckte Element (7) ein Teil eines Mandrins (11) ist.

5. Steuerbare Elektrodenvorrichtung nach einem der Ansprüche 1 bis 4, **dadurch gekennzeichnet**, dass die Kerben (8) V- oder U-förmig ausgebildet sind.

6. Steuerbare Elektrodenvorrichtung nach einem der Ansprüche 1 bis 5, **dadurch gekennzeichnet**, dass das langgestreckte Element (7) elastisch ist, so dass es seine ursprüngliche Form einnimmt, wenn der Druck abnimmt.

7. Steuerbare Elektrodenvorrichtung nach einem der Ansprüche 1 bis 5, **dadurch gekennzeichnet**, dass das langgestreckte Element (7) aus einem derartigen Material besteht, dass es die gebogene Form beibehält, wenn der Druck abnimmt.

8. Steuerbare Elektrodenvorrichtung nach einem der Ansprüche 1 bis 7, **dadurch gekennzeichnet**, dass das langgestreckte Element (7) im Elektrodenkabel (1) drehbar angeordnet ist.

9. Verfahren zur Steuerung der Elektrodenvorrichtung mit Steuermittel nach einem der Ansprüche 1 bis 8, **dadurch gekennzeichnet**, dass das langgestreckte Element (7) mit Hilfe des Mandrins (11) in seine Längsrichtung einem Druck ausgesetzt wird.

10. Verfahren nach Anspruch 9, **dadurch gekennzeichnet**, dass der Druck, dem das langgestreckte Element (7) ausgesetzt wird, von der Biegung des Elektrodenkabels (1), die erreicht werden soll, abhängig ist.

## Claims

1. Controllable electrode device for intracardial stimulation of the heart, having control means and an electrode sable (1) which comprises an extended flexible conductor (2), the outside of which is provided with an insulating layer (3) and the inside of which forms a channel (4) for the insertion of the control means, and having an electrode head (6) which is applied to the distal end of the conductor (2) to stimulate the heart tissue, characterized in that the control means are an elongated element (7) which is provided with at least one indentation (8), which extends approximately at right angles to the longitudinal direction of the element (7) and which has such a form and depth that the element (7) and therefore also the electrode cable (1) are bent in the area of the indentation (8) if the element (7) is subjected to a pressure in its longitudinal direction.

2. Controllable electrode device according to claim 1, characterized in that the elongated element (7) is equipped with a number of successively provided indentations (8) which form a part (9) which is serrated in section.

3. Controllable electrode device according to claim 1 or 2, characterized In that the serrated part (9) abuts the front part of the electrode cable (1).

4. Controllable electrode device according to one of claims 1 to 3, characterized in that the elongated element (7) is a part of a mandrin (11).

5. Controllable electrode device according to one of claims 1 to 4, characterized in that the indentations (8) are constructed to be V-shaped or U-shaped.

6. Controllable electrode device according to one of claims 1 to 5, characterized in that the elongated element (7) is elastic, so that it occupies its original shape if the pressure decreases.

7. Controllable electrode device according to one of claims 1 to 5, characterized in that the elongated element (7) consists of such a material that it maintains the bent shape if the pressure decreases.

8. Controllable electrode device according to one of claims 1 to 7, characterized in that the elongated element (7) is rotatably arranged in the electrode cable (1).

9. Method for controlling the electrode device with control means according to one of claims 1 to 8, characterized in that the elongated element (7) with the aid of the mandrin (11) is subjected to a pressure in its longitudinal direction.

10. Method according to claim 9, characterized in that the pressure to which the elongated element (7) is subjected depends on the bending of the electrode cable (1) which is to be achieved.

## Revendications

1. Dispositif d'électrodes pouvant être commandé pour la stimulation intracardiaque du coeur comportant des moyens de commande et un câble (1) d'électrode, lequel comprend un conducteur (2) souple, oblong, dont le côté extérieur est muni d'une couche (3) isolante et dont le côté intérieur forme un canal (4) pour l'introduction de moyens de commande, et une tête (6) d'électrode, qui est montée à l'extrémité distale du conducteur (2) pour stimuler le tissu cardiaque, caractérisé en ce que les moyens de commande sont un élément (7) oblong, qui est muni d'au moins une encoche (8) sensiblement perpendiculaire à la direction longitudinale de l'élément (7) et qui a une forme et une profondeur telles que l'élément (7), et donc également le câble (1) d'électrode, est courbé dans la région de l'encoche (8), lorsque l'élément (7) subit une pression suivant sa direction longitudinale.

2. Dispositif d'électrodes commandable suivant la revendication 1, caractérisé en ce que l'élément (7) oblong est muni de plusieurs encoches (8) montées les unes à côté des autres, qui forment une partie (9) à profil dentelé.

3. Dispositif d'électrodes commandable suivant la revendication 1 ou 2, caractérisé en ce que la partie (9) dentelée est à la partie avant du câble (1) d'électrode.

4. Dispositif d'électrodes commandable suivant l'une des revendications 1 à 3, caractérisé en ce que l'élément (7) oblong est une partie d'un mandrin (11).

5. Dispositif d'électrodes commandable suivant l'une des revendications 1 à 4, caractérisé en ce que l'encoche (8) est en forme de V ou de U.

6. Dispositif d'électrodes commandable suivant l'une des revendications 1 à 5, caractérisé en ce que l'élément (7) oblong est élastique, de sorte qu'il prenne sa forme initiale lorsque la pression décroît.

7. Dispositif d'électrodes commandable suivant l'une des revendications 1 à 5, caractérisé en ce que l'élément (7) oblong est constitué d'un matériau d'un genre tel qu'il conserve la forme courbée lorsque la pression décroît.

8. Dispositif d'électrodes commandable suivant l'une des revendications 1 à 7, caractérisé en ce que l'élément (7) oblong est disposé rotatif dans le câble (1) d'électrode.

9. Procédé de commande du dispositif d'électrode comportant des moyens de commande suivant l'une des revendications 1 à 8, caractérisé en ce que l'élément (7) oblong est soumis à l'aide du mandrin (11) à une pression suivant sa direction longitudinale.

10. Procédé suivant la revendication 9, caractérisé en ce que la pression, qui est appliquée à l'élément (7) oblong, est fonction de la courbure du câble (1) d'électrode qui doit être atteinte.
